# EUROPEAN PATENT APPLICATION

(11) **EP 1 736 537 A1**
(43) Date of publication of application: **27.12.2006**
(21) Application number: 05013494.9
(22) Date of filing: 22.06.2005
(51) Int. Cl.: C12N 1/20, C12N 1/38, A61K 35/74

(54) **Methods and means for protecting the skin against pathogenic microorganisms**

(71) Applicant: OrganoBalance GmbH, 13355 Berlin (DE)
(72) Inventor: The designation of the inventor has not yet been filed
(74) Representative: Vossius & Partner

(57) **Abstract**

Described are microorganisms which are able to stimulate the growth of microorganisms of the resident skin microbial flora and which do not stimulate the growth of microorganisms of the transient pathogenic micro flora.

Also described are compositions comprising such microorganisms as well as the use of such microorganisms in cosmetic, prophylactic or therapeutic applications.

## Description

The present invention relates to microorganisms which are able to stimulate the growth of microorganisms of the resident skin microbial flora and which do not stimulate the growth of microorganisms of the transient pathogenic micro flora. The present invention also relates to compositions, comprising such microorganisms, e.g. cosmetical or pharmaceutical compositions and to the use of such microorganisms in cosmetic, prophylactic or therapeutic applications.

The human skin is populated by a large variety of microorganisms that mainly live as commensals in a relatively stable composition on the surface of the skin (Roth and James, 1988). This normal skin flora is termed "resident skin flora".
The main function of the human skin is to protect the tissue beneath it against the environment (Feingold, 1985). This normal skin flora especially protects the skin against the intrusion of potentially pathogenic microorganisms (Bisno, 1984). Certain microorganisms dominate the resident microbial flora. More than ninety percent of the microorganisms of the resident microbial flora are *Staphylococcus epidermidis* (coagulase negative), *Micrococcus* spec., Diphteroids and propionibacteria (Leyden et al., 1987). Therefore, a stabilisation of the natural skin flora supports the protection of the skin and prevents the intrusion of pathogens. The health of the skin increases. The importance of the natural skin flora has been described in several clinical studies. It has been shown that in the first days after birth of an infant, where this skin flora has not yet been developed, the danger of a *Staphylococcus aureus* infection is very high. With increasing development of the flora, the skin is protected from the colonization by pathogenic microorganisms (Hurst, 1959). In another study with infants, it has been observed that after treatment with the antibiotic amoxicillin, the resident flora was drastically (about 50%) repressed. This led to more than a fourteen-fold increase of the pathogenic yeast *Candida albicans.* The discontinuation of the antibiotic treatment led to a regeneration of the resident flora and the repression of *Candida albicans* (Brook, 2000).
The microorganisms of the resident skin flora prevent the colonization by pathogenic microorganisms by competing for attachment sites and essential nutrients on the skin surface (Sullivan et al. 2001). Pathogenic microorganisms are able to specifically attach to structures of the epidermis using special binding proteins. In this context, different mechanisms are known. From *Staphylococcus aureus,* for example, specific adhesins are known. These allow the pathogenic microorganism to attach to fibronectin structures. Pathogens generally have a higher potential to attach to the host. This explains the virulence of these microorganisms (Gibbons and Houte, 1975).
The danger of colonization by pathogenic microorganisms increases drastically in the case of small lesions or other damages on the surface of the skin, especially when the normal skin flora is damaged by antibiotics or by excessive washing (Elek, 1956). However, the resident skin flora is better adapted to the skin regarding nutrient utilisation. This leads to an advantage of the resident skin flora (Larson, 2001). Apart from this, the organisms of the resident skin flora are able to produce antimicrobial substances to fight against pathogenic microorganisms. This is also an advantage for resident microorganisms regarding nutrients and energy sources (Selwyn and Ellis, 1972; Milyani and Selwyn, 1978).
Moreover, substances that are secreted by the skin, like complex lipids (triglycerides), are degraded to unsaturated fatty acids that inhibit pathogenic microorganisms like *Streptococcus pyrogenes* or gram negative bacteria and fungi (Aly et al., 1972).
The microbial skin flora affects several factors of the skin that are of cosmetic relevance. These are pH value of the skin, barrier function and lipid content. *S. epidermidis* is able to fight against pathogenic microorganisms by lowering the pH value (about 4-6). Pathogens are not able to grow at decreased pH values (Korting et al., 1990; Lukas, 1990; Korting, 1992; Yosipovitch and Maibach, 1996; Gfatter et al., 1997).
The water barrier function and the lipid content of the skin depend on the ceramide content of the horny layers (Imokawa et al., 1986). Lowering of the ceramide content causes a drying and rifting of the skin. A study with atopical dermatitis patients having these appearances of the skin showed that the microbial skin flora dramatically changes to *Staphylococcus aureus.* This pathogen features a very high ceramidase activity, while normal commensals of the resident skin flora do not have this activity. Sphingomyelinase activities that lead to the release of ceramides in the skin are comparable in the resident and pathogenic flora of atopic dermatitis patients (Ohnishi et al., 1999).

Thus, there is a need for means and methods allowing to protect the skin, in particular the human skin, against pathogenic microorganisms.

The present invention addresses this need and provides microorganisms and methods which protect the skin against the colonization by pathogenic microorganisms. In particular, it provides the embodiments as characterized in the claims.
Accordingly, the present invention in a first aspect relates to a microorganism which is able to stimulate the growth of one or more microorganisms of the resident skin microbial flora and which does not stimulate the growth of microorganisms of the transient pathogenic micro flora.
The inventors surprisingly found that an effective protection of the skin against a colonization by pathogenic microorganisms can be achieved by administering to the skin the above described microorganisms or inactivated forms thereof. The inventors for the first time identified corresponding microorganisms and provided methods for their identification. These microorganisms are able to regenerate and to stabilize the natural skin flora due to a specific stimulation of the growth of microorganisms of the resident skin microbial flora. By this, the growth of pathogenic microorganisms is suppressed. Furthermore, the entrance of pathogenic microorganisms into the skin microbial flora can be prevented. The microorganism of the present invention allow, e.g., to stimulate the resident microbial flora in deeper horny layers of the skin when microorganisms in the upper layers of the skin have been removed by washing.
Many different microorganisms exist on the skin. Some belong to the normal (resident) flora of the skin and are harmless commensals and some are potential pathogens.
Basically, organisms on the skin can be classified into two categories: 1. Resident organisms: resident organisms are permanent inhabitants of the skin which colonise on the surface of the skin, the stratum corneum and within the outer layer of the epidermis and the deeper crevices of the skin and hair follicles. These microorganisms of the resident microbial skin flora can grow and multiply on the skin without invading or damaging the skin tissue. Washing does not easily remove these organisms in deeper skin regions. Resident microorganisms are harmless commensals.
2. Transient organisms: transient organisms are microorganisms which are deposited on the skin but do not multiply there or contaminants which multiply on the skin and persist for short periods. They cannot settle permanently on healthy skin whose microenvironment is heavily determined by the resident micro flora. Transient organisms are potentially pathogenic.

Thus, the term "resident skin microbial flora" relates to the microorganisms which can normally be found on healthy skin, preferably human skin, and which constitute the majority of the microorganisms found on the skin..
In particular, the term "resident skin microbial flora" relates to microorganisms which are permanent inhabitants on the surface of the skin, the stratum corneum and within the outer layer of the epidermis and the deeper crevices of the skin and hair follicles. These microorganisms are characterized in that they can grow and multiply on the skin without invading or damaging the skin tissue. A characteristic of these microorganisms is that washing does not easily remove them in deeper skin regions. The microorganisms of the resident skin microbial flora are harmless commensals.

The term "resident skin microbial flora" preferably relates to a flora of aerobic and anaerobic microorganisms which can be found on skin, preferably human skin. More preferably, it relates to a flora of microorganisms which comprises *Staphylococcus epidermidis* (coagulase negative), *Micrococcus* spec., Diphteroids and propioni bacteria. Typically, about 90 % of the aerobic resident microbial skin flora consists of *Staphylococcus epidermidis.* The remaining about 10 % are composed of mainly *Micrococcus spec.* (80 % *Micrococcus luteus*) and Diphteroids (13 %). The term "Diphtheroid" denotes a wide range of bacteria belonging to the genus *Corynebacterium.* For convenience, cutaneous diphtheroids have been categorized into the following four groups: lipophilic or nonlipophilic diphtheroids; anaerobic diphtheroids; diphtheroids producing porphyrins. Major representatives (90%) of the anaerobic microbial skin flora are propionibacteria; especially *Propionibacterium acnes, P. granulosum* and *P. avidum* can be isolated from the skin. The anaerobic flora accounts for approximately 4 % of the total resident skin flora.

More preferably, more than 90% of the microorganisms of the microbial flora belong to *Staphylococcus epidermidis, Micrococcus* spec., Diphteroids and propioni bacteria. Even more preferably, the resident skin microbial flora is characterized in that its major constituent is *Staphylococcus epidermidis.*
The constituents and the composition of the microbial skin flora can be determined quantitatively and qualitatively, e.g. by peeling off the upper skin layers with scotch tape. Microorganisms of the resident skin microbial flora can be identified within the upper ten skin layers peeled off, e.g., by scotch tape. Exemplary, to isolate these microorganisms six 2 cm² scotch tapes are each pressed on a defined region of the skin, preferably of the forearm and afterwards each tape stripe is transferred from the skin to a selective culture agar plate for either gram positive (e.g. BHI, Difco Inc.) or gram negative bacteria (e.g. MacConkey agar, Difco Inc.) or to a selective culture agar for yeasts and fungi (e.g. Plate Count Agar, Difco Inc.). Afterwards the microorganisms that have been transferred from skin to culture agar plates are cultivated at 30°C and 37°C, aerobically and anaerobically for about 24 hours. Colony forming units are determined by morphological and biochemical methods for a qualitative analysis and by counting for quantification. The relative composition and total cell counts are determined. The person skilled in the art can determine the genus and/or species of the microorganisms of the resident skin microbial flora which have been isolated as described above by methods known in the art. For example, the person skilled in the art may identify said microorganisms due to metabolic footprinting, fatty acid composition and composition of the cell wall etc.

The term "stimulates" in connection with the growth of microorganisms of the resident skin microbial flora means that the growth of one or more of these microorganisms is increased when contacted with a microorganism according to the invention. An increased growth means preferably an increase in proliferation, i.e. cell divisions per time unit. Alternatively, the term "stimulates" also refers to an increase in size of individual cells. Bacterial cell size can be assessed by flow cytometry (e.g. Becton-Dickinson FACSort flow cytometer, San José, CA) after staining with the stain SYBR Green I (Molecular Probes, USA). Bacteria cell size is assessed in Side-Angle Light Scatter (SSC) mode.

An increased growth thus means an increase in biomass production per time unit.

The stimulation of growth of the microorganism(s) of the resident skin microbial flora can preferably be observed in vitro, more preferably in an assay in which a microorganism according to the invention is contacted with one or more microorganisms of the resident skin microbial flora and the growth of the(se) microorganism(s) of the resident skin microbial flora is determined. The growth can be determined by counting the numbers of cells/colonies after different time intervals of incubation and can be compared with a control which does not contain a microorganism according to the invention, thereby allowing to determine whether there is an increase in growth.

An in vitro assay for determining the stimulation of growth is described in the Examples and comprises a so-called "in vitro hole plate assay". In brief, such an assay comprises the following steps:
- cultivation of at least one microorganism of the resident skin microbial flora and evenly spreading it/them on a prepared agar plate containing a suitable agar medium for growth, and preferably detection, of the respective microorganism(s);
- providing holes in the inoculated agar plate;
- filling the holes with precultured cells of a microorganism according to the invention;
- incubating the agar plates for an appropriate amount of time and under conditions allowing growth of the microorganism(s) of the resident skin microbial flora; and
- determining the growth of the microorganism(s) of the resident skin microbial flora surrounding the holes containing a microorganism according to the invention and comparing it to the growth of the microorganism(s) surrounding a hole which does not contain a microorganism according to the invention.
The determination of the growth in the last step may be effected by available means and methods for determining the number of cells and/or colonies, e.g. by staining with an appropriate dye and/or optical means such as densitometry and counting the cells/colonies under the microscope.
Even more preferably, the stimulation of growth of the microorganism(s) of the resident skin microbial flora can also be observed in an in situ skin assay. Such assay is described in the Examples and, in brief, comprises the following steps:
- cultivation of at least one microorganism of the resident skin microbial flora and evenly spreading it on an area of skin of a test individual;
- applying an aliquot of a microorganism according to the invention in a punctual area within the area on which the microorganism(s) of the resident skin microbial flora has/have been spread;
- incubating the skin for an amount of time sufficient to allow growth of the microorganism(s) of the resident skin microbial flora;
- transferring the upper skin layers, including the microorganisms comprised in these, to an agar plate containing an appropriate growth medium;
- incubation of the agar plates for a period of time and under conditions allowing the growth of the microorganism(s) of the resident skin microbial flora;
- determining the growth of the microorganism(s) of the resident skin microbial flora surrounding the area at which the microorganism according to the invention was applied and comparing it to the growth of the microorganism(s) in a control in which no microorganism of the invention was applied.

The area of skin used for this assay may be any suitable area of skin of an individual, preferably of a human individual. In a preferred embodiment it is an area of skin on the forearm of a human individual. The size of the area is not decisive, preferably it is about 1 to 40 cm², more preferably 5 to 20 cm², even more preferably 5 to 10 cm² , e.g. about 5,6, 7, 8, 9 or 10 cm².
The microorganism(s) of the resident skin microbial flora are evenly distributed on the area, preferably in a density of approximately 10² cfu/cm² - 10³ cfu/cm². The microorganism(s) spread on the skin are air dried and an aliquot of a microorganism according to the invention is applied in a punctual manner within the area. This can be achieved by means known to the person skilled in the art. For example, the microorganisms according to the invention are centrifuged (15 min, 4000 x g). The cell pellet is washed two times with K/Na-buffer (each 1 ml). Cells are resuspended in 200 µl K/Na buffer and 10 µl of prepared microorganisms are punctual applied on the pre-inoculated skin area with a micro pipet
The incubation of the skin preferably takes place at room temperature for, e.g., two hours. The transfer of the upper skin layers, including the microorganisms comprised therein, may, e.g., be effected with the help of an adhesive tape stripe. The agar plates to which the upper skin layers have been transferred are incubated at a temperature allowing growth of the microorganism(s) or the resident skin microbial flora to be tested and contain a growth medium known to support growth of this (these) microorganism(s). The incubation typically takes place for about 24 hours.
The growth of the microorganism(s) can be detected by methods known to the person skilled in the art. Preferably, it is determined by densitometry or by counting the colonies formed in the neighborhood of the point at which an aliquot of the microorganism of the invention was applied. Bacterial cell size can be assessed by flow cytometry (e.g. Becton-Dickinson FACSort flow cytometer, San José, CA) after staining with the stain SYBR Green I (Molecular Probes, USA). Bacteria cell size is assessed in Side-Angle Light Scatter (SSC) mode.

A microorganism is regarded to stimulate the growth of one or more microorganisms of the resident skin microbial flora if it leads to an increase of growth of at least one such microorganism in an in vitro hole plate assay of at least 5 %", preferably of at least 10%, 20%, 30%, 40%, 50%, 60%, or 70%, more preferably of at least 75% and even more preferably of at least 80% and most preferably of at least 85% in comparison to a control to which no microorganism has been added.
More preferably, a microorganism is regarded as stimulating the growth of one or more microorganisms of the resident skin microbial flora if it leads to an increase of growth of at least one such microorganism in an in situ skin assay of at least 5 %, preferably of at least 10%, 20%, 30%, 40%, 50%, 60%, or 70%, more preferably of at least 75 %, even more preferably of at least 80 % and most preferably of at least 85 %.

In a preferred embodiment the microorganism according to the invention stimulates the growth of the major representative of the residual skin flora, i.e. *Staphylococcus epidermidis.* The meaning of the word "stimulates growth" is as described herein-above and preferably means a stimulation in vitro, more preferably in an in vitro hole plate assay as described herein-above. Even more preferably it means a stimulation in an in situ skin assay as described herein-above. Most preferably it means a stimulation in an in vitro as well as in an in situ assay. The in vitro hole plate assay and the in situ skin assay are preferably carried out as described in the Examples. In a preferred embodiment the microorganism of the present invention also stimulates the growth of *Micrococcus* spec., preferably of Micrococcus luteus. In a more preferred embodiment, also the growth of Diphteroids, preferably of bacteria belonging to the genus *Corynebacterium* is stimulated.
In a particularly preferred embodiment the microorganism according to the invention stimulates the growth of all microorganisms of the resident skin microbial flora.

The microorganism according to the invention is also characterized in that it does not stimulate the growth of microorganisms of the transient pathogenic micro flora. The term "transient pathogenic micro flora" refers to microorganisms which are deposited on the skin but do not multiply there or to contaminants which multiply on the skin and persist for short periods. In particular, if a microorganism is applied to the skin and is unable to grow and reproduce there under the environmental conditions provided by the healthy skin and cannot permanently colonize this organ (or a region of it), it is considered to belong to the transient pathogenic micro flora. Several bacteria, yeast and fungi can be transiently isolated from human skin but particularly the following microorganism can be classified to the transient micro flora due to their frequent appearance: *Staphylococcus aureus, Streptococcus pyogenes,* gram-negative bacilli (e.g *Acinetobacter calcoaceticus*), *Candida albicans* and *Malassezia furfur.* Microorganisms of the transient micro flora often have pathogenic factors that allow the bacterium to attach to disordered skin regions. This can e.g. be the attachment to collagen structures or keratin structures.
The microorganisms of the transient pathogenic micro flora can be determined, e.g., by metabolic footprinting, the evaluation of fatty acid composition and the composition of the cell wall, sequencing of 16S ribosomal RNA or the detection of specific DNA probes encoding specific pathogenic factors.
The term "does not stimulate the growth of microorganisms of the transient pathogenic micro flora" means that the microorganism of the invention does not stimulate the growth of at least one, preferably of more than one, preferably of more than two, more preferably of more than five and particularly preferred of any of the microorganisms of the transient pathogenic flora.
A microorganism is regarded as not stimulating the growth of a microorganism of the transient pathogenic micro flora if it does not lead to an increased growth of such a microorganism of the transient pathogenic micro flora when contacted with it. The stimulation of growth or its absence can be tested in vitro or in situ as described above in connection with the property of a microorganism of the invention to stimulate the growth of at least one microorganism of the resident skin microbial flora. Most preferably the test for determining stimulation or its absence takes place by carrying out an in vitro hole plate assay and/or an in situ skin assay as described above, more preferably as described in the Examples. A microorganism is regarded as not stimulating the growth of a microorganism of the transient pathogenic micro flora if the growth of the latter microorganism is not increased or only slightly increased when contacted with the former microorganism. "Slightly increased" means that the growth is increased not more than by 5% when compared to the control, more preferably not more than 2% when compared to the control. The term "not increased" means that there can be found no statistically relevant difference between the growth of the microorganism of the transient pathogenic micro flora contacted with a microorganism of the invention when compared to the control where no microorganism of the invention is present. The term "not increased" in a preferred embodiment also includes those cases where a microorganism actually leads to a decrease of the growth of a microorganism of the transient pathogenic micro flora, i.e. where it represses the growth of such a microorganism.
In another preferred embodiment the microorganism of the present invention does not negatively influence the growth of the microorganisms of the transient pathogenic micro flora. The term "not negatively influence" means that that there can be found no inhibition of the growth of the microorganism of the transient pathogenic micro flora contacted with a microorganism of the invention when compared to the control where no microorganism of the invention is present.

In a further preferred embodiment, the microorganism of the present invention does not stimulate the growth of the major representative of the transient pathogenic micro flora, i.e. *Staphylococcus aureus.* The test for determining whether a microorganism does or does not stimulate the growth of *Staphylococcus aureus* is preferably an in vitro and/or an in situ test as described herein-above, more preferably a test as described in the Examples.

In a particularly preferred embodiment the microorganism of the present invention is a microorganism belonging to the group of lactic acid bacteria. The term "microorganism belonging to the group of lactic acid bacteria" encompasses (a) microorganism(s) which belong(s) to bacteria, in particular belonging to gram-positive fermentative eubacteria, more particularly belonging to the family of lactobacteriaceae including lactic acid bacteria. Lactic acid bacteria are from a taxonomical point of view divided up into the subdivisions of Streptococcus, Leuconostoc, Pediococcus and Lactobacillus. The microorganism of the present invention is preferably a Lactobacillus species. Members of the lactic acid bacteria group normally lack porphyrins and cytochromes, do not carry out electron-transport phosphorylation and hence obtain energy only by substrate-level phosphorylation. I.e. in lactic acid bacteria ATP is synthesized through fermentation of carbohydrates. All of the lactic acid bacteria grow anaerobically, however, unlike many anaerobes, most lactic acid bacteria are not sensitive to oxygen and can thus grow in its presence as well as in its absence. Accordingly, the bacteria of the present invention are preferably aerotolerant anaerobic lactic acid bacteria, preferably belonging to the genus of Lactobacillus.
The lactic acid bacteria of the present invention are preferably rod-shaped or spherical, varying from long and slender to short bent rods, are moreover preferably immotile and/or asporogenous and produce lactic acid as a major or sole product of fermentative metabolism. The genus Lactobacillus to which the microorganism of the present invention belongs in a preferred embodiment is divided up by the following characteristics into three major subgroups, whereby it is envisaged that the Lactobacillus species of the present invention can belong to each of the three major subgroups:
(a) homofermentative lactobacilli
   (i) producing lactic acid, preferably the L-, D- or DL-isomer(s) of lactic acid in an amount of at least 85% from glucose via the Embden-Meyerhof pathway;
   (ii) growing at a temperature of 45°C, but not at a temperature of 15°C;
   (iii) being long-rod shaped; and
   (iv) having glycerol teichoic acid in the cell wall;
(b) homofermantative lactobacilli
   (i) producing lactic acid, preferably the L- or DL-isomer(s) of lactic acid via the Embden-Meyerhof pathway;
   (ii) growing at a temperature of 15°C, showing variable growth at a temperature of 45°C;
   (iii) being short-rod shaped or coryneform; and
   (iv) having ribitol and/or glycerol teichoic acid in their cell wall;
(c) heterofermentative lactobacilli
   (i) producing lactic acid, preferably the DL-isomer of lactic acid in an amount of at least 50% from glucose via the pentose-phosphate pathway;
   (ii) producing carbondioxide and ethanol
   (iii) showing variable growth at a temperature of 15°C or 45°C;
   (iv) being long or short rod shaped; and
   (v) having glycerol teichoic acid in their cell wall.

Based on the above-described characteristics, the microorganisms of the present invention can be classified to belong to the group of lactic acid bacteria, particularly to the genus of Lactobacillus. By using classical systematics, for example, by reference to the pertinent descriptions in "Bergey's Manual of Systematic Bacteriology" (Williams & Wilkins Co., 1984), a microorganism of the present invention can be determined to belong to the genus of Lactobacillus. Alternatively, the microorganisms of the present invention can be classified to belong to the genus of Lactobacillus by methods known in the art, for example, by their metabolic fingerprint, i.e. a comparable overview of the capability of the microorganism(s) of the present invention to metabolize sugars or by other methods described, for example, in Schleifer et al., System. Appl. Microb., 18 (1995), 461-467 or Ludwig et al., System. Appl. Microb., 15 (1992), 487-501. The microorganisms of the present invention are capable of metabolizing sugar sources which are typical and known in the art for microorganisms belonging to the genus of Lactobacillus.

The affiliation of the microorganisms of the present invention to the genus of Lactobacillus can also be characterized by using other methods known in the art, for example, using SDS-PAGE gel electrophoresis of total protein of the species to be determined and comparing them to known and already characterized strains of the genus Lactobacillus. The techniques for preparing a total protein profile as described above, as well as the numerical analysis of such profiles, are well known to a person skilled in the art. However, the results are only reliable insofar as each stage of the process is sufficiently standardized. Faced with the requirement of accuracy when determining the attachment of a microorganism to the genus of Lactobacillus, standardized procedures are regularly made available to the public by their authors such as that of Pot et al., as presented during a "workshop" organized by the European Union, at the University of Ghent, in Belgium, on Sep. 12 to 16, 1994 (Fingerprinting techniques for classification and identification of bacteria, SDS-PAGE of whole cell protein). The software used in the technique for analyzing the SDS-PAGE electrophoresis gel is of crucial importance since the degree of correlation between the species depends on the parameters and algorithms used by this software. Without going into the theoretical details, quantitative comparison of bands measured by a densitometer and normalized by a computer is preferably made with the Pearson correlation coefficient. The similarity matrix thus obtained may be organized with the aid of the UPGMA (unweighted pair group method using average linkage) algorithm that not only makes it possible to group together the most similar profiles, but also to construct dendograms (see Kersters, Numerical methods in the classification and identification of bacteria by electrophoresis, in Computer-assisted Bacterial Systematics, 337-368, M. Goodfellow, A. G. O'Donnell Ed., John Wiley and Sons Ltd, 1985).

Alternatively, the affiliation of said microorganisms of the present invention to the genus of Lactobacillus can be characterized with regard to ribosomal RNA in a so called Riboprinter.RTM. More preferably, the affiliation of the newly identified species of the invention to the genus Lactobacillus is demonstrated by comparing the nucleotide sequence of the 16S ribosomal RNA of the bacteria of the invention, or of their genomic DNA which codes for the 16S ribosomal RNA, with those of other genera and species of lactic acid bacteria known to date. Another preferred alternative for determining the attachment of the newly identified species of the invention to the genus Lactobacillus is the use of species-specific PCR primers that target the 16S-23S rRNA spacer region. Another preferred alternative is RAPD-PCR (Nigatu et al. in Antonie van Leenwenhoek (79), 1-6, 2001) by virtue of that a strain specific DNA pattern is generated which allows to determine the affiliation of an identified microorganisms in accordance with the present invention to the genus of Lactobacillus. Further techniques useful for determining the affiliation of the microorganism of the present invention to the genus of Lactobacillus are restriction fragment length polymorphism (RFLP) (Giraffa et al., lnt. J. Food Microbiol. 82 (2003), 163-172), fingerprinting of the repetitive elements (Gevers et al., FEMS Microbiol. Lett. 205 (2001) 31-36) or analysis of the fatty acid methyl ester (FAME) pattern of bacterial cells (Heyrman et al., FEMS Microbiol. Lett. 181 (1991), 55-62). Alternatively, lactobacilli can be determined by lectin typing (Annuk et al., J. Med. Microbiol. 50 (2001), 1069-1074) or by analysis of their cell wall proteins (Gatti et al., Lett. Appl. Microbiol. 25 (1997), 345-348.

In a preferred embodiment of the present application the microorganism is a probiotic Lactobacillus species. The term "probiotic" in the context of the present invention means that the microorganism has a beneficial effect on health if it is topically applied to the skin. Preferably, a "probiotic" microorganism is a live microorganism which, when topically applied to the skin, is beneficial for health of this tissue. Most preferably, this means that the microorganism has a positive effect on the micro flora of the skin.

In a preferred embodiment the microorganism of the present invention belongs to the species of *Lactobacillus paracasei, Lactobacillus brevis* or *Lactobacillus fermentum.* However, the Lactobacillus species are not limited thereto.

In a particularly preferred embodiment of the present invention the microorganism of the present invention is selected from the group consisting of *Lactobacillus paracasei, Lactobacillus brevis* or *Lactobacillus fermentum* being deposited at the DSMZ under the accession number DSM **17248** (*Lactobacillus paracasei* ssp *paracasei* LB-OB-H02), DSM **17247** *(Lactobacillus brevis* LB-OB-H01), DSM **17250** *(Lactobacillus brevis* LB-OB-H04) and DSM **17249** *(Lactobacillus fermentum* LB-OB-H03). The invention also relates to a mutant or derivative of the above-mentioned deposited Lactobacillus strains wherein said mutants or derivatives have retained their capability to stimulate the growth of at least one microorganism of the resident skin microbial flora and their property not to stimulate the growth of microorganisms of the transient pathogenic micro flora.
The term *"Lactobacillus paracasei, Lactobacillus brevis* or *Lactobacillus fermentum* being deposited at the DSMZ under the accession number" relates to cells of a microorganism belonging to the species *Lactobacillus paracasei, Lactobacillus brevis* or *Lactobacillus fermentum* deposited at the Deutsche Sammlung für Mikroorganismen und Zellkulturen (DSMZ) on April 18, 2005 and having the following deposit numbers: DSM 17248 (*Lactobacillus paracasei* ssp *paracasei* LB-OB-H02), DSM **17247** (*Lactobacillus brevis* LB-OB-H01, DSM **17250** (*Lactobacillus brevis* LB-OB-H04) and DSM **17249** (*Lactobacillus fermentum* LB-OB-H03). The DSMZ is located at the Mascheroder Weg 1 b, D-38124 Braunschweig, Germany. The aforementioned deposits were made pursuant to the terms of the Budapest treaty on the international recognition of the deposit of microorganisms for the purposes of patent procedures.

In a particular preferred embodiment the microorganisms of the present invention are "isolated" or "purified". The term "isolated" means that the material is removed from its original environment, e.g. the natural environment if it is naturally occurring, or the culture medium if it is cultured. For example, a naturally-occurring microorganism, preferably a Lactobacillus species, separated from some or all of the coexisting materials in the natural system, is isolated. Such a microorganism could be part of a composition, and is to be regarded as still being isolated in that the composition is not part of its natural environment.
The term "purified" does not require absolute purity; rather, it is intended as a relative definition. Individual microorganisms obtained from a library have been conventionally purified to microbiological homogeneity, i.e. they grow as single colonies when streaked out on agar plates by methods known in the art. Preferably, the agar plates that are used for this purpose are selective for Lactobacillus species. Such selective agar plates are known in the art.

In another aspect the present invention relates to an inactivated form of the microorganism of the present invention, which is, e.g., thermally inactivated or lyophilized, but which retains the property of stimulating the growth of microorganisms of the resident skin microbial flora and of not stimulating the growth of microorganisms of the transient pathogenic micro flora.
According to the present invention the term "inactivated form of the microorganism of the present invention" includes a dead or inactivated cell of the microorganism of the present invention, preferably of the Lactobacillus species disclosed herein, which is no longer capable to form a single colony on a plate specific for microorganisms belonging to the genus of Lactobacillus. Said dead or inactivated cell may have either an intact or broken cell membrane. Methods for killing or inactivating cells of the microorganism of the present invention are known in the art. EI-Nezami et al., J. Food Prot. 61 (1998), 466-468 describes a method for inactivating Lactobacillus species by UV-irradiation. Preferably, the cells of the microorganism of the present invention are thermally inactivated or lyophilised. Lyophilisation of the cells of the present invention has the advantage that they can be easily stored and handled while retaining their property to stimulate growth of microorganisms of the resident skin microbial flora while not stimulating the growth of microorganisms of the transient pathogenic micro flora. Moreover, lyophilised cells can be grown again when applied under conditions known in the art to appropriate liquid or solid media. Lyophilization is done by methods known in the art. Preferably, it is carried out for at least 2 hours at room temperature, i.e. any temperature between 16°C and 25°C. Moreover, the lyophilized cells of the microorganism of the present invention are stable for at least 4 weeks at a temperature of 4°C so as to still retain their properties as described above. Thermal inactivation can be achieved by incubating the cells of the microorganism of the present invention for at least 2 hours at a temperature of 170°C. Yet, thermal inactivation is preferably achieved by autoclaving said cells at a temperature of 121 °C for at least 20 minutes in the presence of satured steam at an atmospheric pressure of 2 bar. In the alternative, thermal inactivation of the cells of the microorganism of the present invention is achieved by freezing said cells for at least 4 weeks, 3 weeks, 2 weeks, 1 week, 12 hours, 6 hours, 2 hours or 1 hour at -20°C. It is preferred that at least 70%, 75% or 80%, more preferably 85%, 90% or 95% and particularly preferred at least 97%, 98%, 99% and more particularly preferred, 99.1%, 99.2%, 99.3%, 99.4%, 99.5%, 99.6%, 99.7%, 99.8% or 99.9% and most particularly preferred 100% of the cells of the inactivated form of the microorganism of the present invention are dead or inactivated, however, they have still the capability to stimulate growth of microorganisms of the resident skin microbial flora but do not stimulate growth of microorganisms of the transient pathogenic micro flora. Whether the inactivated form of the microorganism of the present invention is indeed dead or inactivated can be tested by methods known in the art, for example, by a test for viability.

The term "inactivated form of the microorganism of the present invention" also encompasses lysates or fractions of the microorganism of the present invention, preferably of the Lactobacillus species disclosed herein, wherein said lysates or fractions preferably stimulate the growth of a microorganism of the resident skin microbial flora and does not stimulate the growth of microorganisms of the transient pathogenic micro flora, in particular, *Staphylococcus aureus* as described herein. This stimulation can be tested as described herein and in particular as described in the appended Examples. In case, a lysate or fraction of the microorganism of the present invention may stimulate the growth of a microorganism of the transient pathogenic micro flora, then the skilled person can, for example, further purify said lysate or fraction by methods known in the art, which are exemplified herein below, so as to remove substances which may stimulate the growth of microorganisms of the transient pathogenic micro flora. Afterwards the person skilled in the art can again test said lysate or fraction whether it stimulates the growth of a microorganism of the resident skin microbial flora but not the growth of a microorganism of the transient pathogenic micro flora.
According to the present invention the term "lysate" means a solution or suspension in an aqueous medium of cells of the microorganism of the present invention that are broken. However, the term should not be construed in any limiting way. The cell lysate comprises, e.g., macromolecules, like DNA, RNA, proteins, peptides, carbohydrates, lipids and the like and/or micromolecules, like amino acids, sugars, lipid acids and the like, or fractions of it. Additionally, said lysate comprises cell debris which may be of smooth or granular structure. Methods for preparing cell lysates of microorganism are known in the art, for example, by employing French press, cells mill using glass or iron beads or enzymatic cell lysis and the like. In addition, lysing cells relates to various methods known in the art for opening/destroying cells. The method for lysing a cell is not important and any method that can achieve lysis of the cells of the microorganism of the present invention may be employed. An appropriate one can be chosen by the person skilled in the art, e.g. opening/destruction of cells can be done enzymatically, chemically or physically. Non-limiting examples for enzymes and enzyme cocktails are proteases, like proteinase K, lipases or glycosidases; non-limiting examples for chemicals are ionophores, detergents, like sodium dodecyl sulfate, acids or bases; and non-limiting examples of physical means are high pressure, like French-pressing, osmolarity, temperature, like heat or cold. Additionally, a method employing an appropriate combination of an enzyme other than the proteolytic enzyme, an acid, a base and the like may also be utilized. For example, the cells of the microorganism of the present invention are lysed by freezing and thawing, more preferably freezing at temperatures below -70°C and thawing at temperatures of more than 30°C, particularly freezing is preferred at temperatures below -75°C and thawing is preferred at temperatures of more than 35°C and most preferred are temperatures for freezing below -80°C and temperatures for thawing of more than 37°C. It is also preferred that said freezing/thawing is repeated for at least 1 time, more preferably for at least 2 times, even more preferred for at least 3 times, particularly preferred for at least 4 times and most preferred for at least 5 times.
Accordingly, those skilled in the art can prepare the desired lysates by referring to the above general explanations, and appropriately modifying or altering those methods, if necessary. Preferably, the aqueous medium used for the lysates as described is water, physiological saline, or a buffer solution. An advantage of a bacterial cell lysate is that it can be easily produced and stored cost efficiently since less technical facilities are needed.
According to the invention, lysates are also preparations of fractions of molecules from the above-mentioned lysates. These fractions can be obtained by methods known to those skilled in the art, e.g., chromatography, including, e.g., affinity chromatography, ionexchange chromatography, size-exclusion chromatography, reversed phase-chromatography, and chromatography with other chromatographic material in column or batch methods, other fractionation methods, e.g., filtration methods, e.g., ultrafiltration, dialysis, dialysis and concentration with size-exclusion in centrifugation, centrifugation in density-gradients or step matrices, precipitation, e.g., affinity precipitations, salting-in or salting-out (ammoniumsulfate-precipitation), alcoholic precipitations or other proteinchemical, molecular biological, biochemical, immunological, chemical or physical methods to separate above components of the lysates. In a preferred embodiment those fractions which are more immunogenic than others are preferred. Those skilled in the art are able to choose a suitable method and determine its immunogenic potential by referring to the above general explanations and specific explanations in the examples herein, and appropriately modifying or altering those methods, if necessary.
The term "an inactive form of the microorganism of the present invention" encompasses any part of the cells of the microorganism of the present invention. Preferably, said inactive form is a membrane fraction obtained by a membrane-preparation. Membrane preparations of microorganisms belonging to the genus of Lactobacillus can be obtained by methods known in the art, for example, by employing the method described in Rollan et al., Int. J. Food Microbiol. 70 (2001), 303-307, Matsuguchi et al., Clin. Diagn. Lab. Immunol. 10 (2003), 259-266 or Stentz et al., Appl. Environ. Microbiol. 66 (2000), 4272-4278 or Varmanen et al., J. Bacteriology 182 (2000), 146-154. Alternatively, a whole cell preparation is also envisaged.

In another aspect the present invention relates to a composition comprising a microorganism according to the present invention or a mutant, derivative or inactive form of this microorganism as described above. In a preferred embodiment, said composition comprises a microorganism as described above in an amount between 10² to 10¹² cells, preferably 10³ to 10⁸ cells per mg in a solid form of the composition. In case of a liquid form of compositions, the amount of the microorganisms is between 10² to 10¹³ cells per ml. In a further preferred embodiment said compositions are in the form of emulsions, e.g. oil in water or water in oil emulsions, in the form of ointments or in the form of microcapsules. In case of emulsions, ointments or microcapsules the compositions comprise a microorganism as described herein in an amount between 10² to 10¹³ cells per ml. However, for specific compositions the amount of the microorganism may be different as is described herein.

In a still further aspect, the present invention provides a method for the production of a composition for protecting the skin against pathogenic microorganisms comprising the steps of formulating a microorganism according to the invention or a mutant, derivative or inactive form of this microorganism as described above with a cosmetically or pharmaceutical acceptable carrier or excipient.

The term "composition", as used in accordance with the present invention, relates to (a) composition(s) which comprise(s) at least one microorganism of the present invention or mutant, derivative or inactive form of said microorganism as described above. It is envisaged that the compositions of the present invention which are described herein below comprise the aforementioned ingredients in any combination. It may, optionally, comprise at least one further ingredient suitable for protecting the skin against pathogenic microorganisms. Accordingly, it may optionally comprise any combination of the hereinafter described further ingredients. The term "ingredients suitable for protecting the skin against pathogenic microorganisms" encompasses compounds or compositions and/or combinations thereof which lower the pH.

The composition may be in solid, liquid or gaseous form and may be, inter alia, in the form of (a) powder(s), (a) solution(s) (an) aerosol(s), suspensions, emulsions, liquids, elixirs, extracts, tincture or fluid extracts or in a form which is particularly suitable for topical administration. Forms suitable for topical application include, e.g., a paste, an ointment, a lotion, a cream, a gel or a transdermal patch.

Preferably, the composition of the present invention is a cosmetic composition further comprising a cosmetically acceptable carrier or excipient. More preferably, said cosmetic composition is a paste, an ointment, a lotion, a cream or a gel.

The cosmetic composition of the present invention comprises the microorganism of the present invention, mutant, derivative or inactive form thereof as described above in connection with the composition of the invention and further a cosmetically acceptable carrier. Preferably the cosmetic composition of the present invention is for use in topical applications.
The term "cosmetically acceptable carrier" as used herein means a suitable vehicle, which can be used to apply the present compositions to the skin in a safe and effective manner. Such vehicle may include materials such as emulsions, e.g. oil in water or water in oil emulsions, ointments or micro capsules. It is also advantageous to administer the active ingredients in encapsulated form, e.g. as cellulose encapsulation, in gelatine, with polyamides, niosomes, wax matrices, with cyclodextrins or liposomally encapsulated. The term "safe and effective amount" as used herein, means a sufficient amount to stimulate growth of at least one microorganism of the resident skin microbial flora.

In another aspect the present invention relates to a pharmaceutical composition comprising the microorganism of the present invention or a derivative or mutant or an inactive form thereof as described above further comprising a pharmaceutical acceptable carrier or excipient. The pharmaceutical composition preferably is in a form which is suitable for topical administration.

In addition, the present invention relates to the use of a microorganism of the present invention or of a derivative or mutant or an inactive form thereof as described above for the preparation of a composition, preferably a pharmaceutical or cosmetic composition.

Pharmaceutical compositions comprise a therapeutically effective amount of a microorganism of the present invention or of a derivative or mutant of the present invention or an inactive form of said microorganism of the present invention as described above and can be formulated in various forms, e.g. in solid, liquid, powder, aqueous, lyophilized form.
The pharmaceutical composition may be administered with a pharmaceutically acceptable carrier to a patient, as described herein. In a specific embodiment, the term "pharmaceutically acceptable" means approved by a regulatory agency or other generally recognized pharmacopoeia for use in animals, and more particularly in humans.

The term "carrier" refers to a diluent, adjuvant, excipient, or vehicle with which the therapeutic is administered. Such a carrier is pharmaceutically acceptable, i.e. is non-toxic to a recipient at the dosage and concentration employed. It is preferably isotonic, hypotonic or weakly hypertonic and has a relatively low ionic strength, such as provided by a sucrose solution. Such pharmaceutical carriers can be sterile liquids, such as water and oils, including those of petroleum, animal, vegetable or synthetic origin, such as peanut oil, soybean oil, mineral oil, sesame oil and the like. Saline solutions and aqueous dextrose and glycerol solutions can also be employed as liquid carriers. Suitable pharmaceutical excipients include starch, glucose, sucrose, gelatin, malt, rice, flour, chalk, silica gel, sodium stearate, glycerol monostearate, talc, sodium ion, dried skim milk, glycerol, propylene, glycol, water, ethanol and the like. The composition, if desired, can also contain minor amounts of wetting or emulsifying agents, or pH buffering agents. These compositions can take the form of, e.g., solutions, suspensions, emulsion, powders, sustained-release formulations and the like. Examples of suitable pharmaceutical carriers are described in "Remington's Pharmaceutical Sciences" by E.W. Martin. Some other examples of substances which can serve as pharmaceutical carriers are sugars, such as glucose and sucrose; starches such as corn starch and potato starch; cellulose and its derivatives such as sodium carboxymethycellulose, ethylcellulose and cellulose acetates; powdered tragancanth; malt; gelatin; talc; stearic acids; magnesium stearate; calcium sulfate; calcium carbonate; vegetable oils, such as peanut oils, cotton seed oil, sesame oil, olive oil, corn oil and oil of theobroma; polyols such as propylene glycol, glycerine, sorbitol, manitol, and polyethylene glycol; agar; alginic acids; pyrogen-free water; isotonic saline; cranberry extracts and phosphate buffer solution; skim milk powder; as well as other non-toxic compatible substances used in pharmaceutical formulations such as Vitamin C, estrogen and echinacea, for example. Wetting agents and lubricants such as sodium lauryl sulfate, as well as coloring agents, flavoring agents, lubricants, excipients, tabletting agents, stabilizers, anti-oxidants and preservatives, can also be present. It is also advantageous to administer the active ingredients in encapsulated form, e.g. as cellulose encapsulation, in gelatine, with polyamides, niosomes, wax matrices, with cyclodextrins or liposomally encapsulated.
Generally, the ingredients are supplied either separately or mixed together in unit dosage form, for example, as a dry lyophilised powder or water free concentrate in a hermetically sealed container such as an ampoule or sachette indicating the quantity of active agent.
The pharmaceutical composition of the invention can be formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.
In vitro or in situ assays, e.g. those described in the Examples, may optionally be employed to help identify optimal dosage ranges. The precise dose to be employed in the formulation will also depend on the route of administration, and the seriousness of the disease or disorder, and should be decided according to the judgment of the practitioner and each patient's circumstances. The topical route of administration is preferred. Effective doses may be extrapolated from dose-response curves derived from in vitro or (animal) model test systems. Preferably, the pharmaceutical composition is administered directly or in combination with an adjuvant. Adjuvants may be selected from the group consisting of a chloroquine, protic polar compounds, such as propylene glycol, polyethylene glycol, glycerol, EtOH, 1-methyl L-2-pyrrolidone or their derivatives, or aprotic polar compounds such as dimethylsulfoxide (DMSO), diethylsulfoxide, di-n-propylsulfoxide, dimethylsulfone, sulfolane, dimethylformamide, dimethylacetamide, tetramethylurea, acetonitrile or their derivatives. These compounds are added in conditions respecting pH limitations. The composition of the present invention can be administered to a vertebrate. "Vertebrate" as used herein is intended to have the same meaning as commonly understood by one of ordinary skill in the art. Particularly, "vertebrate" encompasses mammals, and more particularly humans.
The term "administered" means administration of a therapeutically effective dose of the aforementioned composition. By "therapeutically effective amount" is meant a dose that produces the effects for which it is administered, preferably this effect is the protection of skin against pathogenic microorganisms. The exact dose will depend on the purpose of the treatment, and will be ascertainable by one skilled in the art using known techniques. As is known in the art and described above, adjustments for systemic versus localized delivery, age, body weight, general health, sex, diet, time of administration, drug interaction and the severity of the condition may be necessary, and will be ascertainable with routine experimentation by those skilled in the art.
The methods are applicable to both human therapy and veterinary applications. The compounds described herein having the desired therapeutic activity may be administered in a physiologically acceptable carrier to a patient, as described herein. Depending upon the manner of administration, the compounds may be formulated in a variety of ways as discussed below. The concentration of the therapeutically active compound in the formulation may vary from about 0.01-100 wt %. The agent may be administered alone or in combination with other treatments.
The administration of the pharmaceutical composition can be done in a variety of ways. The preferable route of administering is the topical route.
The attending physician and clinical factors will determine the dosage regimen. As is well known in the medical arts, dosages for any one patient depends upon many factors, including the patient's size, body surface area, age, the particular compound to be administered, sex, time and route of administration, general health, and other drugs being administered concurrently. A typical dose can be, for example, in the range of 0.001 to 1000 µg; however, doses below or above this exemplary range are envisioned, especially considering the aforementioned factors.
The dosages are preferably given once a week, more preferably 2 times, 3 times, 4 times, 5 times or 6 times a week and most preferably daily and even more preferably, 2 times a day or more often. In particular, it may be preferable to give a dosage each time after a disturbance of the resident skin flora occurred, e.g. by washing. However, during progression of the treatment the dosages can be given in much longer time intervals and in need can be given in much shorter time intervals, e.g., several times a day. In a preferred case the immune response is monitored using herein described methods and further methods known to those skilled in the art and dosages are optimized, e.g., in time, amount and/or composition. Progress can be monitored by periodic assessment. It is also envisaged that the pharmaceutical compositions are employed in co-therapy approaches, i.e. in co-administration with other medicaments or drugs, for example other drugs for protecting skin against pathogenic microorganisms.

Topical administration of the cosmetic or pharmaceutical composition of the present invention is useful when the desired treatment involves areas or organs readily accessible by topical administration. For application topically to the skin, the pharmaceutical composition is preferably formulated with a suitable paste, ointment, lotion, cream, gel or transdermal patches. The cosmetic or pharmaceutical preparations can, depending on the field of use, also be in the form of a spray (pump spray or aerosol), foam, gel spray, mousse, suspensions or powders.

A suitable paste comprises the active ingredient suspended in a carrier. Such carriers include, but are not limited to, petroleum, soft white paraffin, yellow petroleum jelly and glycerol.
The cosmetic or pharmaceutical composition may also be formulated with a suitable ointment comprising the active components suspended or dissolved in a carrier. Such carriers include, but are not limited to, one or more of glycerol, mineral oil, liquid oil, liquid petroleum, white petroleum, yellow petroleum jelly, propylene glycol, alcohols, triglycerides, fatty acid esters such as cetyl ester, polyoxyethylene polyoxypropylene compound, waxes such as white wax and yellow beeswax, fatty acid alcohols such as cetyl alcohol, stearyl alcohol and cetylstearylalcohol, fatty acids such as stearic acid, cetyl stearate, lanolin, magnesium hydroxide, kaolin and water.
Alternatively, the cosmetic or pharmaceutical composition may also be formulated with a suitable lotion or cream comprising the active components suspended or dissolved in a carrier. Such carriers include, but are not limited to, one or more of mineral oil such as paraffin, vegetable oils such as castor oil, castor seed oil and hydrogenated castor oil, sorbitan monostearat, polysorbat, fatty acid esters such as cetyl ester, wax, fatty acid alcohols such as cetyl alcohol, stearyl alcohol, 2-octyldodecanol, benzyl alcohol, alcohols, triglycerides and water.
Alternatively, the cosmetic or pharmaceutical composition may also be formulated with a suitable gel comprising the active components suspended or dissolved in a carrier. Such carriers include, but are not limited to, one or more of water, glycerol, propyleneglycole, liquid paraffin, polyethylene, fatty oils, cellulose derivatives, bentonite and colloidal silicon dioxide.

Suitable propellants for aerosols according to the invention are the customary propellants, for example propane, butane, pentane and others.

The preparations according to the invention may generally comprise further auxiliaries as are customarily used in such preparations, e.g. preservatives, perfumes, antifoams, dyes, pigments, thickeners, surface-active substances, emulsifiers, emollients, finishing agents, fats, oils, waxes or other customary constituents, of a cosmetic or dermatological formulation, such as alcohols, polyols, polymers, foam stabilizers, solubility promoters, electrolytes, organic acids, organic solvents, or silicone derivatives.

The cosmetic or pharmaceutical composition according to the invention may comprise emollients. Emollients may be used in amounts which are effective to prevent or relieve dryness. Useful emollients include, without limitation: hydrocarbon oils and waxes; silicone oils; triglyceride esters; acetoglyceride esters; ethoxylated glyceride; alkyl esters; alkenyl esters; fatty acids; fatty alcohols; fatty alcohol ethers; etheresters; lanolin and derivatives; polyhydric alcohols (polyols) and polyether derivatives; polyhydric alcohol (polyol) esters; wax esters; beeswax derivatives; vegetable waxes; phospholipids; sterols; and amides.
Thus, for example, typical emollients include mineral oil, especially mineral oils having a viscosity in the range of 50 to 500 SUS, lanolin oil, mink oil, coconut oil, cocoa butter, olive oil, almond oil, macadamia nut oil, aloa extract, jojoba oil, safflower oil, corn oil, liquid lanolin, cottonseed oil, peanut oil, purcellin oil, perhydrosqualene (squalene), caster oil, polybutene, odorless mineral spirits, sweet almond oil, avocado oil, calophyllum oil, ricin oil, vitamin E acetate, olive oil, mineral spirits, cetearyl alcohol (mixture of fatty alcohols consisting predominantly of cetyl and stearyl alcohols), linolenic alcohol, oleyl alcohol, octyl dodecanol, the oil of cereal germs such as the oil of wheat germ cetearyl octanoate (ester of cetearyl alcohol and 2-ethylhexanoic acid), cetyl palmitate, diisopropyl adipate, isopropyl palmitate, octyl palmitate, isopropyl myristate, butyl myristate, glyceryl stearate, hexadecyl stearate, isocetyl stearate, octyl stearate, octylhydroxy stearate, propylene glycol stearate, butyl stearate, decyl oleate, glyceryl oleate, acetyl glycerides, the octanoates and benzoates of (C12-C15) alcohols, the octanoates and decanoates of alcohols and polyalcohols such as those of glycol and glycerol, and ricin- oleates of alcohols and poly alcohols such as those of isopropyl adipate, hexyl laurate, octyl dodecanoate, dimethicone copolyol, dimethiconol, lanolin, lanolin alcohol, lanolin wax, hydrogenated lanolin, hydroxylated lanolin, acetylated lanolin, petrolatum, isopropyl lanolate, cetyl myristate, glyceryl myristate, myristyl myristate, myristyl lactate, cetyl alcohol, isostearyl alcohol stearyl alcohol, and isocetyl lanolate, and the like.

Moreover, the cosmetic or pharmaceutical composition according to the invention may also comprise emulsifiers. Emulsifiers (i.e., emulsifying agents) are preferably used in amounts effective to provide uniform blending of ingredients of the composition. Useful emulsifiers include (i) anionics such as fatty acid soaps, e.g., potassium stearate, sodium stearate, ammonium stearate, and triethanolamine stearate; polyol fatty acid monoesters containing fatty acid soaps, e.g., glycerol monostearate containing either potassium or sodium salt; sulfuric esters (sodium salts), e.g., sodium lauryl 5 sulfate, and sodium cetyl sulfate; and polyol fatty acid monoesters containing sulfuric esters, e.g., glyceryl monostearate containing sodium lauryl surfate; (ii) cationics chloride such as N(stearoyl colamino formyl methyl) pyridium; N-soya-N-ethyl morpholinium ethosulfate; alkyl dimethyl benzyl ammonium chloride; diisobutylphenoxytheoxyethyl dimethyl benzyl ammonium chloride; and cetyl pyridium chloride; and (iii) nonionics such as polyoxyethylene fatty alcohol ethers, e.g., monostearate; polyoxyethylene lauryl alcohol; polyoxypropylene fatty alcohol ethers, e.g., propoxylated oleyl alcohol; polyoxyethylene fatty acid esters, e.g., polyoxyethylene stearate; polyoxyethylene sorbitan fatty acid esters, e.g., polyoxyethylene sorbitan monostearate; sorbitan fatty acid esters, e.g., sorbitan; polyoxyethylene glycol fatty acid esters, e.g., polyoxyethylene glycol monostearate; and polyol fatty acid esters, e.g., glyceryl monostearate and propylene glycol monostearate; and ethoxylated lanolin derivatives, e.g., ethoxylated lanolins, ethoxylated lanolin alcohols and ethoxylated cholesterol. The selection of emulsifiers is exemplarly described in Schrader, Grundlagen und Rezepturen der Kosmetika, Hüthig Buch Verlag, Heidelberg, 2nd edition, 1989, 3^{rd} part.
The cosmetic or pharmaceutical composition according to the invention may also include a surfactant. Suitable surfactants may include, for example, those surfactants generally grouped as cleansing agents, emulsifying agents, foam boosters, hydrotropes, solubilizing agents, suspending agents and nonsurfactants (facilitates the dispersion of solids in liquids).

The surfactants are usually classified as amphoteric, anionic, cationic and nonionic surfactants. Amphoteric surfactants include acylamino acids and derivatives and N-alkylamino acids. Anionic surfactants include: acylamino acids and salts, such as, acylglutamates, acylpeptides, acylsarcosinates, and acyltaurates; carboxylic acids and salts, such as, alkanoic acids, ester carboxylic acids, and ether carboxylic acids; sulfonic acids and salts, such as, acyl isethionates, alkylaryl sulfonates, alkyl sulfonates, and sulfosuccinates; sulfuric acid esters, such as, alkyl ether sulfates and alkyl sulfates. Cationic surfactants include: alkylamines, alkyl imidazolines, ethoxylated amines, and quaternaries (such as, alkylbenzyldimethylammonium salts, alkyl betaines, heterocyclic ammonium salts, and tetra alkylammonium salts). And nonionic surfactants include: alcohols, such as primary alcohols containing 8 to 18 carbon atoms; alkanolamides such as alkanolamine derived amides and ethoxylated amides; amine oxides; esters such as ethoxylated carboxylic acids, ethoxylated glycerides, glycol esters and derivatives, monoglycerides, polyglyceryl esters, polyhydric alcohol esters and ethers, sorbitan/sorbitol esters, and triesters of phosphoric acid; and ethers such as ethoxylated alcohols, ethoxylated lanolin, ethoxylated polysiloxanes, and propoxylated polyoxyethylene ethers.

Furthermore, a cosmetic or pharmaceutical composition according to the invention may also comprise a film former. Suitable film formers which are used in accord with the invention keep the composition smooth and even and include, without limitation: acrylamide/sodium acrylate copolymer; ammonium acrylates copolymer; Balsam Peru; cellulose gum; ethylene/maleic anhydride copolymer; hydroxyethylcellulose; hydroxypropylcellulose; polyacrylamide; polyethylene; polyvinyl alcohol; pvm/MA copolymer (polyvinyl methylether/maleic anhydride); PVP (polyvinylpyrrolidone); maleic anhydride copolymer such as PA-18 available from Gulf Science and Technology; PVP/hexadecene copolymer such as Ganex V-216 available from GAF Corporation; acryliclacrylate copolymer; and the like.
Generally, film formers can be used in amounts of about 0.1 % to about 10% by weight of the total composition with about 1% to about 8% being preferred and about 0.1 DEG/O to about 5% being most preferred. Humectants can also be used in effective amounts, including: fructose; glucose; glulamic acid; glycerin; honey; maltitol; methyl gluceth-10; methyl gluceth-20; propylene glycol; sodium lactate; sucrose; and the like.

Of course, the cosmetic or pharmaceutical composition of the present invention can also comprise a preservative. Preservatives according to certain compositions of the invention include, without limitation: butylparaben; ethylparaben; imidazolidinyl urea; methylparaben; O-phenylphenol; propylparaben; quaternium-14; quaternium-15; sodium dehydroacetate; zinc pyrithione; and the like.
The preservatives are used in amounts effective to prevent or retard microbial growth. Generally, the preservatives are used in amounts of about 0.1 % to about 1% by weight of the total composition with about 0.1% to about 0.8% being preferred and about 0.1% to about 0.5% being most preferred.

A cosmetic or pharmaceutical composition according to the invention may also comprise a perfume. Perfumes (fragrance components) and colorants (coloring agents) well known to those skilled in the art may be used in effective amounts to impart the desired fragrance and color to the compositions of the invention.

Furthermore, a cosmetic or pharmaceutical composition of the present invention may also comprise a wax. Suitable waxes which are useful in accord with the invention include: animal waxes, such as beeswax, spermaceti, or wool wax (lanolin); plant waxes, such as carnauba or candelilla; mineral waxes, such as montan wax or ozokerite; and petroleum waxes, such as paraffin wax and microcrystalline wax (a high molecular weight petroleum wax). Animal, plant, and some mineral waxes are primarily esters of a high molecular weight fatty alcohol with a high molecular weight fatty acid. For example, the hexadecanoic acid ester of tricontanol is commonly reported to be a major component of beeswax. Other suitable waxes according to the invention include the synthetic waxes including polyethylene polyoxyethylene and hydrocarbon waxes derived from carbon monoxide and hydrogen.
Representative waxes also include: cerosin; cetyl esters; hydrogenated joioba oil; hydrogenated jojoba wax; hydrogenated rice bran wax; Japan wax; jojoba butter; jojoba oil; jojoba wax; munk wax; montan acid wax; ouricury wax; rice bran wax; shellac wax; sufurized jojoba oil; synthetic beeswax; synthetic jojoba oils; trihydroxystearin; cetyl alcohol; stearyl alcohol; cocoa butter; fatty acids of lanolin; mono-, di- and 25 triglycerides which are solid at 25 DEG C., e.g., glyceyl tribehenate (a triester of behenic acid and glycerine) and C1g-C36 acid triglyceride (a mixture of triesters of C1g-C36 carboxylic acids and glycerine) available from Croda, Inc., New York, N.Y. under the tradenames Syncrowax HRC and Syncrowax HGL-C, respectively; fatty esters which are solid at 25 DEG C.; silicone waxes such as methyloctadecaneoxypolysiloxane and poly (dimethylsiloxy) stearoxysiloxane; stearyl mono- and diethanolamide; rosin and its derivatives such as the abietates of glycol and glycerol; hydrogenated oils solid at 25 DEG C.; and sucroglycerides. Thickeners (viscosity control agents) which may be used in effective amounts in aqueous systems include: algin; carbomers such as carbomer 934, 934P, 940 and 941; cellulose gum; cetearyl alcohol, cocamide DEA, dextrin; gelatin; hydroxyethylcellulose; hydroxypropylcellulose; hydroxypropyl methylcellulose; magnesium aluminum silicate; myristyl alcohol; oat flour; oleamide DEA; oleyl alcohol; PEG-7M; PEG-14M; PEG-90M; stearamide DEA; stearamide MEA; stearyl alcohol; tragacanth gum; wheat starch; xanthan gum; and the likein the above list of thickeners, DEA is diethanolamine, and MEA is monoethanolamine. Thickeners (viscosity control agents) which may be used in effective amounts in nonaqueous systems include aluminum stearates; beeswax; candelilla wax; carnauba; ceresin; cetearyl alcohol; cetyl alcohol; cholesterol; hydrated silica; hydrogenated castor oil; hydrogenated cottonseed oil; hydrogenated soybean oil; hydrogenated tallow glyceride; hydrogenated vegetable oil; hydroxypropyl cellulose; lanolin alcohol; myristyl alcohol; octytdodecyl stearoyl sulfate; oleyl alcohol; ozokerite; microcystalline wax; paraffin, pentaerythrityl tetraoctanoate; polyacrylamide; polybutene; polyethylene; propylene glycol dicaprylate; propylene glycol dipelargonate; stearalkonium hectorite; stearyl alcohol; stearyl stearate; synthetic beeswax; trihydroxystearin; trilinolein; tristearin; zinc stearate; and the like.
Customary native and synthetic thickeners or gel formers in formulations are crosslinked polyacrylic acids and derivatives thereof, polysaccharides, such as xanthane gum or alginates, carboxymethylcellulose or hydroxycarboxymethylcellulose, hydrocolloids such as gum Arabic or montmorillonite minerals, such as bentonites or fatty alcohols, polyvinyl alcohol and polyvinlypyrrolidone.

Other ingredients which can be added or used in a cosmetic or pharmaceutical composition according to the invention in amounts effective for their intended use, include: biological additives to enhance performance or consumer appeal such as amino acids, proteins, vanilla, aloe extract, bioflavinoids, and the like; buffering agents, chelating agents such as EDTA; emulsion stabilizers; pH adjusters; opacifying agents; and propellants such as butane carbon clioxide, ethane, hydrochlorofluorocarbons 22 and 142b, hydrofluorocarbon 152a, isobutane, isopentane, nitrogen, nitrous oxide, pentane, propane, and the like.

Furthermore, the preparations according to the invention may also comprise compounds which have an antioxidative, free-radical scavenger, skin moisturizing or moisture-retaining, antierythematous,antiinflammatory or antiallergic action, in order to supplement or enhance their action. In particular, these compounds can be chosen from the group of vitamins, plant extracts, alpha- and beta-hydroxy acids, ceramides, antiinflammatory, antimicrobial or UV-filtering substances, and derivatives thereof and mixtures thereof. Advantageously, preparations according to the invention can also comprise substances which absorb UV radiation in the UV-B and/or UV-A region. The lipid phase is advantageously chosen from the group of substances of mineral oils, mineral waxes, branched and/or unbranched hydrocarbons and hydrocarbon waxes, triglycerides of saturated and/or unsaturated, branched and/or unbranched C.sub.8-C.sub.24-alkanecarboxylic acids; they can be chosen from synthetic, semisynthetic or natural oils, such as olive oil, palm oil, almond oil or mixtures; oils, fats or waxes, esters of saturated and/or unsaturated, branched and/or unbranched C.sub.3-C.sub.30-alkane carboxylic acids and saturated and/or unsaturated, branched and/or unbranched C.sub.3-C.sub.30-alcohols, from aromatic carboxylic acids and saturated and/or unsaturated, branched and/or unbranched C.sub.3-C.sub.30-alcohols, for example isopropyl myristate, isopropyl stearate, hexyldecyl stearate, oleyl oleate; and also synthetic, semisynthetic and natural mixtures of such esters, such as jojoba oil, alkyl benzoates or silicone oils, such as, for example, cyclomethicone, dimethylpolysiloxane, diethylpolysiloxane, octamethylcyclotetrasiloxane and mixtures thereof or dialkyl ethers.

The active ingredients according to the invention may, for example, be used in cosmetic compositions for the cleansing of the skin, such as bar soaps, toilet soaps, curd soaps, transparent soaps, luxury soaps, deodorizing soaps, cream soaps, baby soaps, skin protection soaps, abrasive soaps, syndets, liquid soaps, pasty soaps, soft soaps, washing pastes, liquid washing, showering and bath preparations, e.g. washing lotions, shower preparations, shower gels, foam baths, cream foam baths, oil baths, bath extracts, scrub preparations, in-situ products, shaving foams, shaving lotions, shaving creams. In addition, they are suitable for skin cosmetic preparations, such as W/O or O/W skin and body creams, day and night creams, light protection compositions, aftersun products, hand care products, face creams, multiple emulsions, gelees, microemulsions, liposome preparations, niosome preparations, antiwrinkle creams, face oils, lipogels, sportgels, moisturizing creams, bleaching creams, vitamin creams, skin lotions, care lotions, ampoules, aftershave lotions, preshaves, humectant lotions, tanning lotions, cellulite creams, depigmentation compositions, massage preparations, body powders, face tonics, deodorants, antiperspirants, nose strips, antiacne compositions, repellents and others.

In a preferred embodiment, a cosmetic composition comprises a daily care O/W formulation, which may contain, for example, the following ingredients in % in accordance with the International Nomenclature of Cosmetic Ingredients, INCl:

| | | |
|---|---|---|
| A | 1.7 | ceteareth-6, stearyl alcohol |
| | 0.7 | ceteareth-25 |
| | 2.0 | diethylamino hydroxybenzoyl hexyl benzoate |
| | 2.0 | PEG-14 dimethicone |
| | 3.6 | cetearyl alcohol |
| | 6.0 | ethylhexyl methoxycinnamate |
| | 2.0 | dibutyl adipate |
| B | 5.0 | glycerol |
| | 0.2 | disodium EDTA |
| | 1.0 | panthenol |
| | q.s. | preservative |
| | 67.8 | aqua dem. |
| C | 4.0 | caprylic/capric triglyceride, sodium acrylates copolymer |
| D | 0.2 | sodium ascorbyl phosphate |
| | 1.0 | tocopheryl acetate |
| | 0.2 | bisabolol |
| | 1.0 | caprylic/capric triglyceride, sodium ascorbate, tocopherol, retinol |
| | 1.0 | *Lactobacillus spec.* |
| E | q.s. | sodium hydroxide |

Phases A and B are separately heated to app. 80°C. Phase B is subsequently stirred into phase A and homogenized. Phase C is stirred into a combination of phases A and B and homogenized. The mixture is under agitation cooled down to app. 40°C; then phase D is added and the pH is adjusted with phase E to approx. 6.5. The solution is subsequently homogenized and cooled down to room temperature.
In a further preferred embodiment, a cosmetic composition comprises a protecting day cream O/W formulation, which may contain, for example, the following ingredients in % in accordance with the International Nomenclature of Cosmetic Ingredients, INCI:

| | | |
|---|---|---|
| A | 1.7 | ceteareth-6, stearyl alcohol |
| | 0.7 | ceteareth-25 |
| | 2.0 | diethylamino hydroxybenzoyl hexyl benzoate |
| | 2.0 | PEG-14 dimethicone |
| | 3.6 | cetearyl alcohol |
| | 6.0 | ethylhexyl methoxycinnamate |
| | 2.0 | dibutyl adipate |
| B | 5.0 | glycerol |
| | 0.2 | disodium EDTA |
| | 1.0 | panthenol |
| | q.s. | preservative |
| | 68.6 | aqua dem. |
| C | 4.0 | caprylic/capric triglyceride, sodium acrylates copolymer |
| D | 1.0 | sodium ascorbyl phosphate |
| | 1.0 | tocopheryl acetate |
| | 0.2 | bisabolol |
| | 1.0 | *Lactobacillus spec.* |
| E | q.s. | sodium hydroxide |

Phases A and B are separately heated to app. 80°C. Phase B is subsequently stirred into phase A and homogenized. Phase C is introduced into a combination of phases A and B and homogenized. The mixture is under agitation cooled down to app. 40°C; then phase D is added and the pH is adjusted with phase E to about 6.5. The solution is subsequently homogenized and cooled down to room temperature.
In a further preferred embodiment, a cosmetic composition comprises a skin cleanser O/W formulation, which may contain, for example, the following ingredients in % in accordance with the International Nomenclature of Cosmetic Ingredients, INCl:

| | | |
|---|---|---|
| A | 10.0 | cetearyl ethylhexanoate |
| | 10.0 | caprylic/capric triglyceride |
| | 1.5 | cyclopentasiloxane, cyclohexasilosane |
| | 2.0 | PEG-40 hydrogenated castor oil |
| B | 3.5 | caprylic/capric triglyceride, sodium acrylates copolymer |
| C | 1.0 | tocopheryl acetate |
| | 0.2 | bisabolol |
| | q.s. | preservative |
| | q.s. | perfume oil |
| D | 3.0 | polyquaternium-44 |
| | 0.5 | cocotrimonium methosulfate |
| | 0.5 | ceteareth-25 |
| | 2.0 | panthenol, propylene glycol |
| | 4.0 | propylene glycol |
| | 0.1 | disodium EDTA |
| | 1.0 | *Lactobacillus spec.* |
| | 60.7 | aqua dem. |

Initially, phase A is dissolved and phase B subsequently stirred into phase A. Subsequently, phase C is introduced into the combination of phases A and B. In a next step, phase D is dissolved and stirred into combined phases A, B and C. The mixture is homogenized and stirred for 15 min.
In a further preferred embodiment, a cosmetic composition comprises a daily care body spray formulation, which may contain, for example, the following ingredients in % in accordance with the International Nomenclature of Cosmetic Ingredients, INCl:

| | | |
|---|---|---|
| A | 3.0 | ethylhexyl methoxycinnamate |
| | 2.0 | diethylamino hydroxybenzoyl hexyl benzoate |
| | 1.0 | polyquaternium-44 |
| | 3.0 | propylene glycol |
| | 2.0 | panthenol, propylene glycol |
| | 1.0 | cyclopentasiloxane, cyclohexasiloxane |
| | 10.0 | octyldodecanol |
| | 0.5 | PVP |
| | 10.0 | caprylic/capric triglyceride |
| | 3.0 | C12-15 alkyl benzoate |
| | 3.0 | glycerol |
| | 1.0 | tocopheryl acetate |
| | 0.3 | bisabolol |
| | 1.0 | *Lactobacillus spec.* |
| | 59.2 | alcohol |

The components of phase A are weighed out and dissolved until clearness. In a further preferred embodiment, a cosmetic composition comprises a skin gel, which may contain, for example, the following ingredients in % in accordance with the International Nomenclature of Cosmetic Ingredients, INCl:

| | | |
|---|---|---|
| | 3.6 | PEG-40 hydrogenated castor oil |
| | 15.0 | alcohol |
| | 0.1 | bisabolol |
| | 0.5 | tocopheryl acetate |
| | q.s. | perfume oil |
| B | 3.0 | panthenol |
| | 0.6 | carbomer |
| | 1.0 | *Lactobacillus spec.* |
| | 75.4 | aqua dem, |
| C | 0.8 | triethanolamine |

Initially, phase A is dissolved until clearness. Phase B is macerated and subsequently neutralized with phase C. In a next step, phase A is stirred into the homogenized phase B and the mixture is homogenized.
In yet a further preferred embodiment, a cosmetic composition comprises an after shave lotion, which may contain, for example, the following ingredients in % in accordance with the International Nomenclature of Cosmetic Ingredients, INCI:

| | | |
|---|---|---|
| A | 10.0 | cetearyl ethylhexanoate |
| | 5.0 | tocopheryl acetate |
| | 1.0 | bisabolol |
| | 0.1 | perfume oil |
| | 0.3 | acrylates/c10-30 alkyl acrylate crosspolymer |
| B | 15.0 | alcohol |
| | 1.0 | panthenol |
| | 3.0 | glycerol |
| | 1.0 | *Lactobacillus spec.* |
| | 0.1 | triethanolamine |
| | 63.5 | aqua dem. |

The component of phase A are mixed. In a next step, phase B is dissolved and introduced

into phase A and subsequently homogenized.

The present invention also relates to the use of a microorganism according to the invention or of a derivative, mutant or inactive form thereof as described herein above for the preparation of a pharmaceutical composition for preventing or treating dermatitis, preferably atopic dermatitis, psoriasis, poison-ivy dermatitis, eczema herpeticum, kerion or scabies.

In another aspect the present invention relates to a method for the production of a composition comprising the step of formulating a microorganism of the invention or a derivative or mutant thereof or an inactive form as described herein above with a cosmetically and/or pharmaceutically carrier or excipient.

The present invention furthermore relates to a method of preventing or treating dermatitis, preferably atopic dermatitis, psoriasis, poison-ivy dermatitis, eczema herpeticum, kerion or scabies comprising the step of administering to a patient in need thereof a prophylactically or therapeutically effective amount of a composition according to the invention.

It is to be understood that this invention is not limited to the particular methodology, protocols, bacteria, vectors, and reagents etc. described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.
Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).
Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step.

Several documents are cited throughout the text of this specification. Each of the documents cited herein (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention.
It must be noted that as used herein and in the appended claims, the singular forms "a", "an", and "the", include plural referents unless the context clearly indicates otherwise. Thus, for example, reference to "a reagent" includes one or more of such different reagents, and reference to "the method" includes reference to equivalent steps and methods known to those of ordinary skill in the art that could be modified or substituted for the methods described herein.

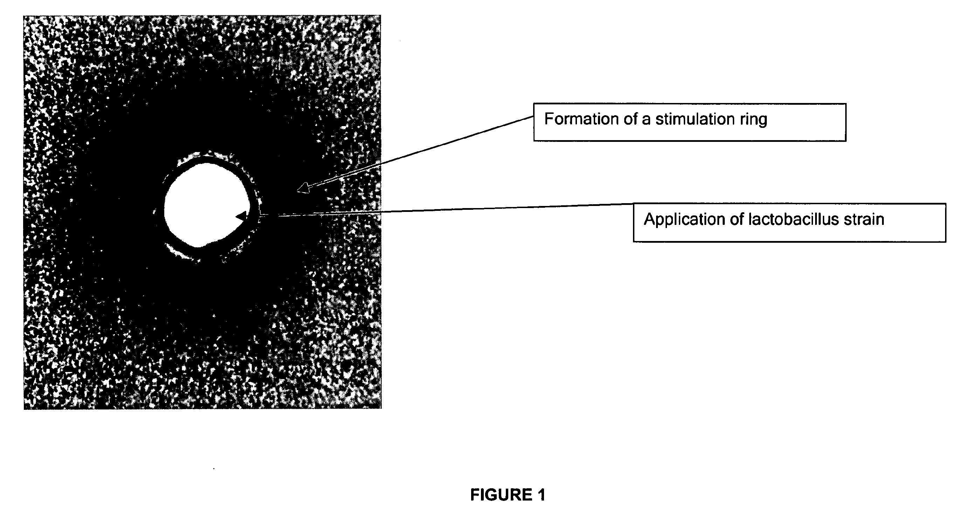

**Figure 1** shows the growth stimulation of Staphylococcus epidermidis in an *in-vitro-*hole/well plate assay (Example 1). The formation of a black ring around the well indicates growth stimulation of the indicator strain Staphylococcus epidermidis. Microscopically an increased number of colonies can be observed.

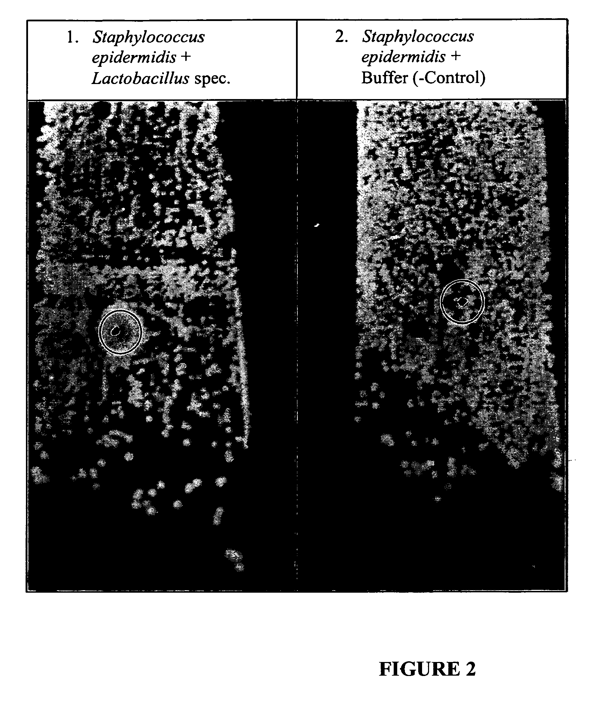

**Figure 2** shows stimulation of Staphylococcus epidermidis on the skin by lactobacilli. Shown are agar plates with the indicator strain *Staphylococcus epidermidis* and a lactobacillus strain that both have been applied to the skin. The upper skin layer has been transferred to an agar plate using an adhesive tape. By this the indicator strain has been transferred to the agar plate. The control plate does not contain the *Lactobacillus* strain.

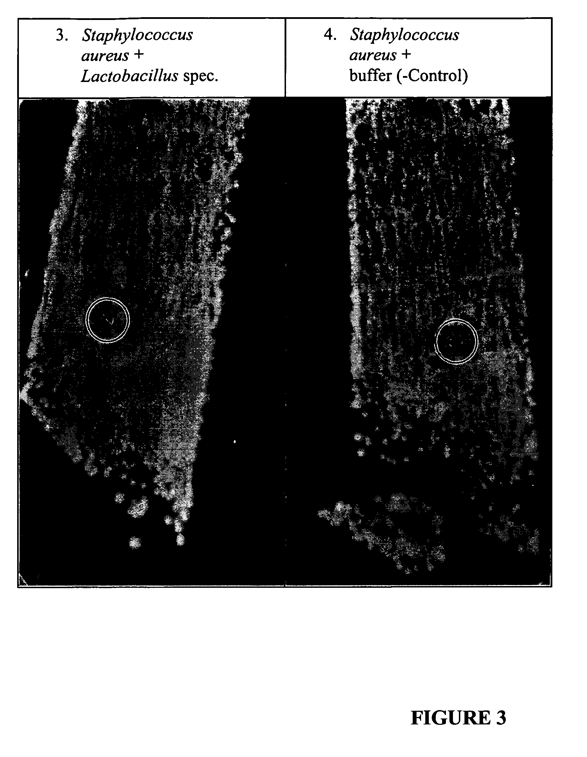

**Figure 3** shows the lack of stimulation of *Staphylococcus aureus* on the skin by lactobacilli. Shown are agar plates with the indicator strain *Staphylococcus aureus* and a lactobacillus strain that both have been applied to the skin. The upper skin layer has been transferred to an agar plate using an adhesive tape. By this the indicator strain has been transferred to the agar plate. The control plate does not contain the *lactobacillus* strain.

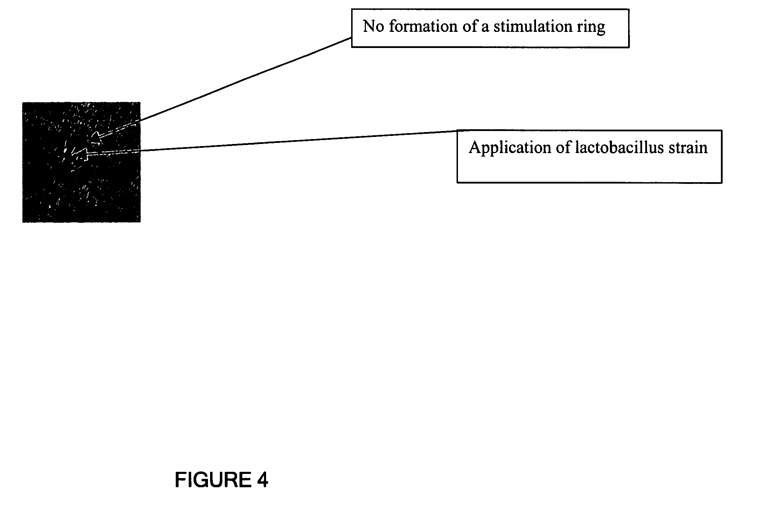

**Figure 4** shows the lack of stimulation of *Staphylococcus aureus* in an in-vitro-hole/well plate assay (Example 4). No formation of a black ring with increased cell density around the well can be observed. This indicates that the indicator strain is not stimulated by the lactobacillus.

### Example 1

### Growth stimulation of S. epidermidis in an in-vitro- hole plate assay

Specific lactic acid bacteria have been identified that are able to stimulate the growth of *Staphylococcus epidermidis* on agar plates in an *in-vitro-* hole plate assay. These lactic acid bacteria are described herein. To test this effect, precultured lactic acid bacteria have been filled into pre-cutted holes and a growth stimulation of the Indicator strain S. *epidermidis* has been observed. To advance the visual effect of growth stimulation Tellurite has been used. Tellurite specifically stains staphylococci. Stimulance was defined as the formation of a black ring around the hole the lactic acid bacterium was pipetted in and an increase of the colony count. Data are shown in Figure 1.

### Cultivation and preparation of lactobacilli:

Lactic acid bacteria were cultivated from a -80°C freezing culture in 1 ml MRS broth in Eppendorf tubes. The tubes were closed and cultivated for 2 days at 37°C. 10 µl of this preculture were transferred to the main culture consisting of 7 ml MRS broth in Falcon tubes. The culture was incubated for two days. After cultivation cells were harvested by centrifugation (15 min, 4000 x g). The cell pellet was washed two times with K/Na-buffer (1 ml each). The cells were resuspended in 200 µl K/Na buffer.

### Cultivation and preparation of the indicator strain:

The indicator strain was *Staphylococcus epidermidis* (DSM20044). 20 ml BHI broth in a shaking glass flask were inoculated with 15 µl of a 24 h preculture. The indicator strain was cultivated for 24 h at 37°C. An aliquot was diluted to an optical density OD₅₉₅ₙₘ of 0.025 - 0.05 in BHI-broth and 800 µl were spread on indicator plates (BHI/Tellurite). The agar was stamped using a cork borer. The holes were filled with the pre cultured lactic acid bacteria.

### Media and buffer:

| | | |
|---|---|---|
| BHI-Agar | Difco Agar 1.8%; 20 ml per plate | |
| BHI-Medium | Difco | |
| BHI/Tellurite-Agar | like BHI-Agar, after cooling to 50°C 1 ml of a sterile filtered 1% potassium-Tellurite solution are transferred to 100 ml BHI-Medium; 20 ml per plate | |
| MRS-broth | Difco, 150 µl/well | |
| K/Na-buffer | Küster Thiel, pH 7.0, autoclaved | |
| | - 0.066 M Na₂HPO₄ x 2H₂O | 61.2 ml |
| | - 0.066 M KH₂PO₄ | 38.8 ml |

### Example 2

### Growth stimulation of Staphylococcus epidermidis in an in-situ- skin assay

Probiotic lactic acid bacteria have been identified that are able to stimulate the growth of *Staphylococcus epidermidis* directly on the skin.
A culture of *Staphylococcus epidermidis* was diluted and directly applied to the skin and air dried. Afterwards an aliquot of the lactic acid bacterium was applied punctual on this skin area. The indicator strain *Staphylococcus epidermidis* can be stimulated directly on the skin by the lactic acid bacterium. After incubation the staphylococci were transferred from the skin to an agar plate using an adhesive tape. The agar plate was incubated at 37°C. An increased colony count indicates a growth stimulation of the indicator strain on the skin (Figure 2). The lactobacilli strains of the present invention, in particular those deposited with the DSMZ exhibited growth stimulation of the indicator strain as described herein.

### Cultivation and preparation of lactobacilli:

Lactic acid bacteria were cultivated from a -80°C freezing culture in 1 ml MRS broth in Eppendorf tubes. The tubes were closed and cultivated for 2 days at 37°C. 10 µl of this preculture were transferred to the main culture consisting of 7 ml MRS broth in Falcon tubes. The culture was incubated for two days. After cultivation cells were harvested by centrifugation (15 min, 4000 x g). The cell pellet was washed two times with K/Na-buffer (1 ml each). The cells were resuspended in 200 µl K/Na buffer.

### Cultivation and preparation of the indicator strain:

The indicator strain was *Staphylococcus epidermidis* (DSM20044). 20 ml BHI broth in a shaking glass flask were inoculated with 15 µl of a 24 h preculture. The indicator strain was cultivated for 24 h at 37°C. An aliquot was diluted to an optical density OD₅₉₅ₙₘ of 0.025 - 0.05 in BHI-broth. This solution was diluted again (1:100).

### Media and buffer:

| | | |
|---|---|---|
| BHI-Agar | Difco Agar 1.8%; 20 ml per plate | |
| BHI-Medium | Difco | |
| MRS-broth | Difco, 150 µl/well | |
| K/Na-buffer | Küster Thiel, pH 7.0, autoclaved | |
| | - 0.066 M Na₂HPO₄ x 2H₂O | 61.2 ml |
| | - 0.066 M KH₂PO₄ | 38.8 ml |

### Application of S. epidermidis on the forearm:

400 µl of a 1:100 dilution of the prepared indicator strain *Staphylococcus epidermidis* was spread evenly on a defined skin area (10 cm x 3 cm) and air dried.

### Application of lactobacilli on the S. epidermidis inoculated skin area:

10 µl of prepared lactobacilli were punctually applied to the *S. epidermidis* pre-inoculated skin area. The arm was incubated for two hours in a normal environment.

### Reisolation of microorganisms from the skin:

After 2 h the four upper skin layers were transferred to a BHI-agar plate using adhesive tape stripes. By this the isolated skin bacteria were transferred to the agar plate. The agar plates were incubated for 24 h at 37°C.

### Example 3

### No growth stimulation of Staphylococcus aureus in an in-situ- skin assay

Using this assay it is possible to check whether unwanted bacteria of the transient, pathogenic microbial flora are not stimulated by lactic acid bacteria that are able to stimulate bacteria of the protecting resident skin microbial flora.

For this purpose the indicator strain *Staphylococcus aureus* was highly diluted and applied to the skin in the same manner as *Staphylococcus epidermidis* (see Example 2). Again the stimulating activity of lactic acid bacteria was tested. A stimulation of *Staphylococcus aureus* by the described lactic acid bacteria could not be observed. The lactobacilli strains of the present invention, in particular those deposited with the DSMZ, did not show stimulation of *Staphylococcus aureus.* Data are presented in Figure 3.

### Cultivation and preparation of lactobacilli:

Lactic acid bacteria were cultivated from a -80°C freezing culture in 1 ml MRS broth in Eppendorf tubes. The tubes were closed and cultivated for 2 days at 37°C. 10 µl of this preculture were transferred to the main culture consisting of 7 ml MRS broth in Falcon tubes. The culture was incubated for two days. After cultivation cells were harvested by centrifugation (15 min, 4000 x g). The cell pellet was washed two times with K/Na-buffer (1 ml each). The cells were resuspended in 200 µl K/Na buffer.

### Cultivation and preparation of the indicator strain:

The indicator strain was *Staphylococcus aureus* (DSM346). 20 ml BHI broth in a shaking glass flask were inoculated with 15 µl of a 24 h preculture. The indicator strain was cultivated for 24 h at 37°C. An aliquot was diluted to an optical density OD₅₉₅ₙₘ of 0.025 - 0.05 in BHI-broth. This solution was diluted again (1:100).

### Media and buffer:

| | | |
|---|---|---|
| BHI-Agar | Difco Agar 1.8%; 20 ml per plate | |
| BHI-Medium | Difco | |
| MRS-broth | Difco, 150 µl/well | |
| K/Na-buffer | Küster Thiel, pH 7.0, autoclaved | |
| | - 0.066 M Na₂HPO₄ x 2H₂O | 61.2 ml |
| | - 0.066 M KH₂PO₄ | 38.8 ml |

### Application of Staphylococcus aureus on the forearm:

400 µl of a 1:100 dilution of the prepared indicator strain *Staphylococcus aureus* was spread evenly on a defined skin area (10 cm x 3 cm) and air dried.

### Application of lactobacilli on the S. aureus inoculated skin area:

10 µl of prepared lactobacilli were punctually applied to the *S. aureus* pre-inoculated skin area. The arm was incubated for two hours in a normal environment.

### Reisolation of microorganisms from the skin:

After 2 h the four upper skin layers were transferred to a BHI-agar plate using adhesive tape stripes. By this the isolated skin bacteria were transferred to the agar plate. The agar plates were incubated for 24 h at 37°C. The data are shown in Figure 3.

### Example 4

### No growth stimulation of S. aureus in an in-vitro- hole plate assay

Specific lactic acid bacteria have been identified that are able to stimulate the growth of *Staphylococcus epidermidis* on agar plates in an *in-vitro-* hole plate assay but not the representative of the transient microbial skin flora *Staphylococcus aureus.* To test this effect, precultured lactic acid bacteria that are able to stimulate *Staphylococcus epidermidis* have been filled into pre-cutted holes and absence of growth stimulation of the indictator strain S. *aureus* has been observed. To advance the visual effect of growth stimulation tellurite has been used. Tellurite specifically stains staphylococci. Stimulance was defined as the formation of a black ring around the hole containing the lactic acid bacterium and an increase of the colony count. The lactobacilli strains of the present invention, in particular those deposited with the DSMZ did not show stimulation of *Staphylococcus aureus.* Data are shown in Figure 4.

### Cultivation and preparation of lactobacilli:

Lactic acid bacteria were cultivated from a -80°C freezing culture in 1 ml MRS broth in Eppendorf tubes. The tubes were closed and cultivated for 2 days at 37°C. 10 µl of this preculture were transferred to the main culture consisting of 7 ml MRS broth in Falcon tubes. The culture was incubated for two days. After cultivation cells were harvested by centrifugation (15 min, 4000 x g). The cell pellet was washed two times with K/Na-buffer (1 ml each). Cells were resuspended in 200 µl K/Na buffer.

### Cultivation and preparation of the indicator strain:

The indicator strain was *Staphylococcus aureus* (DSM346). 20 ml BHI broth in a shaking glass flask were inoculated with 15 µl of a 24 h preculture. The indicator strain was cultivated for 24 h at 37°C. An aliquot was diluted to an optical density OD₅₉₅ₙₘ of 0.025 - 0.05 in BHI-broth and 800 µl were spread on indicator plates (BHI/Tellurite). The agar was stamped using a cork borer. The holes were filled with the pre cultured lactic acid bacteria.

### Media and buffer:

| | | |
|---|---|---|
| BHI-Agar | Difco Agar 1.8%; 20 ml per plate | |
| BHI-Medium | Difco | |
| BHI/Tellurite-Agar | like BHI-Agar, after cooling to 50°C 1 ml of a filter sterilized 1 % potassium-Tellurite solution are transferred to 100 ml BHI-Medium; 20 ml are distributed per plate | |
| MRS-broth | Difco, 150 µl/well | |
| K/Na-buffer | Küster Thiel, pH 7,0, autoclaved | |
| | - 0.066 M Na₂HPO₄ x 2H₂O | 61.2 ml |
| | - 0.066 M KH₂PO₄ | 38.8 ml |

### Cited References

Aly, R., Maibach, HI., Shinefield, HR., Strauss, WG. (1972): Survival of pathogenic microorganisms on human skin. J Invest Dermatol. 58(4): 205-210.
Bisno, AL. (1984): Cutaneous infections: microbiologic and epidemiologic considerations. Am J Med. 76(5A): 172-179.
Brook, I. (2000): The effects of amoxicillin therapy on skin flora in infants. Pediatr Dermatol. 17(5): 360-363.
Elek, SD. (1956): Experimental staphylococcal infections in the skin of man. Ann. NY Acad Sci. 65: 85-90.
Feingold, DS. (1985): Cutaneous microbial flora. Cutis. 36(5A): 1.
Gfatter, R., Hackl, P., Braun, F. (1997): Effects of soap and detergents on skin surface pH, stratum corneum hydration and fat content in infants. Dermatology. 195(3): 258-262.
Gibbons, RJ., Houte, JV. (1975): Bacterial adherence in oral microbial ecology. Annu Rev Microbiol. 1975;29: 19-44.
Hurst, V. (1959): Transmission of hospital staphylococci among newborn infants. Pediatrics 25: 204-214.
Imokawa, G., Akasaki, S., Hattori, M., Yoshizuka, N. (1986): Selective recovery of deranged water-holding properties by stratum corneum lipids. J Invest Dermatol. 87(6): 758-761.
Korting, HC. (1992): Einfluß des pH-Wertes auf das Wachstum von Staphylococcus epidermidis, Staphylococcus aureus und Propionibacterium acnes in kontinuierlicher Kultur. Zbl. Hyg. 193: 78-90.
Korting, HC., Hübner, K., Greiner, K., Hamm, G., Braun-Falco, O. (1990): Unterschiede des Hautoberflächen-pH-Wertes und der bakteriellen Mikroflora durch Langzeit-Anwendung synthetische Detergenz-Zubereitungen mit pH 5,5 und pH 7,0 in Acta Derm Venereol. 70: 429-457.
Larson, E. (2001): Hygiene of the skin: when is clean too clean? Emerg Infect Dis. 7(2): 225-230.
Leyden, JJ., McGinley, KJ., Nordstrom, KM., Webster, GF. (1987): Skin microflora. J Invest Dermatol. 88(3): 65-72.
Lukas, A. (1990): Beeinflußbarkeit des Wachstums wichtiger Bakterien der Residentflora in-vitro durch den pH-Wert. In: O. Braun-Falco, HC. Korting (Hrsg.): Hautreinigung mit Syndets, 104-112.
Milyani, RM., Selwyn, S. (1978): Quantitative studies on competitive activities of skin bacteria growing on solid media. J Med Microbiol. 11 (4): 379-386.
Ohnishi, Y., Okino, N., Ito, M., Imayama, S. (1999): Ceramidase activity in bacterial skin flora as a possible cause of ceramide deficiency in atopic dermatitis. Clin Diagn Lab Immunol. 6(1): 101-104.
Roth, RR., James, WD. (1988): Microbial ecology of the skin. Annu Rev Microbiol. 42: 441-464.
Selwyn, S., Ellis, H. (1972): Skin bacteria and skin disinfection reconsidered. Br Med J. 1(793): 136-140.
Sullivan, A., Edlund, C., Nord, CE. (2001): Effect of antimicrobial agents on the ecological balance of human micro flora. Lancet Infect Dis. 1(2): 101-114.
Yosipovitch, G., Maibach, Hl. (1996): Skin surface pH: A protective acid mantle in Cosmetics Toiletries magazine 111 (12): 101

## Claims

1. A microorganism which is able to stimulate the growth of one or more microorganisms of the resident skin microbial flora and which does not stimulate the growth of microorganisms of the transient pathogenic micro flora.

2. The microorganism of claim 1 which is able to stimulate the growth of *Staphylococcus epidermidis.*

3. The microorganism of claim 2 which is able to stimulate growth of *Staphylococcus epidermidis* in vitro.

4. The microorganism of claim 2 or 3 which is able to stimulate growth of *Staphylococcus epidermidis* in an in situ skin assay.

5. The microorganism of any one of claims 1 to 4 which does not stimulate the growth of *Staphylococcus aureus.*

6. The microorganism of any one of claims 1 to 5 which is a microorganism belonging to the genus of *Lactobacillus.*

7. The microorganism of claim 6, wherein said *Lactobacillus* is *Lactobacillus paracasei, Lactobacillus brevis* or *Lactobacillus fermentum.*

8. The microorganism of claim 7, wherein the *Lactobacillus paracasei* is of the subspecies *Lactobacillus paracasei ssp. paracasei.*

9. The microorganism of claim 7 or 8 which is selected from the group consisting of Lactobacillus paracasei, Lactobacillus brevis or Lactobacillus fermentum having DSMZ accession number DSMZ 17248, accession number DSMZ 17247, accession number DSMZ 17250 and accession number DSMZ 17249 or a mutant or derivative thereof, wherein said mutant or derivative retains the ability to stimulate the growth of at least one microorganism of the resident skin microbial flora and does not stimulate the growth of microorganisms of the transient pathogenic micro flora.

10. An inactive form of the microorganism of any one of claims 1 to 9, which is able to stimulate the growth of one or more microorganisms of the resident skin microbial flora but which does not stimulate the growth of microorganisms of the transient pathogenic micro flora.

11. The inactive form of claim 10, which is thermally inactivated or lyophilized

12. A composition comprising a microorganism of any one of claims 1 to 9 or an inactive form of claim 10 or 11.

13. The composition of claim 12 which is a cosmetic composition optionally comprising a cosmetically acceptable carrier or excipient.

14. The composition of claim 12 which is a pharmaceutical composition optionally comprising a pharmaceutically acceptable carrier or excipient.

15. Use of a microorganism of any one of claims 1 to 9 or of an inactive form of claim 10 or 11 for the preparation of a cosmetic or pharmaceutical composition for protecting skin against pathogenic bacteria.

16. Use of a microorganism of any one of claims 1 to 9 or of an inactive form of claim 10 or 11 for the preparation of a pharmaceutical composition for the prophylaxis or treatment of dermatitis.

17. The use of claim 16, wherein the dermatitis is atopic dermatitis, psoriasis, poison-ivy dermatitis, eczema herpeticum, kerion or scabies.

18. A method for the production of a composition comprising the step of formulating a microorganism according to any one of claims 1 to 9 or an inactive form of claim 10 or 11 with a cosmetically or pharmaceutically acceptable carrier or excipient.
